# EUROPÄISCHE PATENTSCHRIFT

(11) **EP 2 721 002 B1**
(45) Veröffentlichungstag und Bekanntmachung des Hinweises auf die Patenterteilung: **28.11.2018**
(21) Anmeldenummer: 12726419.0
(22) Anmeldetag: 05.06.2012
(51) Int. Cl.: C07C 253/10, C07C 255/46

(54) **VERFAHREN ZUR HERSTELLUNG VON 3-CYANO-3,5,5-TRIMETHYLCYCLOHEXANON**
PROCESS FOR PREPARING 3-CYANO-3,5,5-TRIMETHYLCYCLOHEXANONE
PROCÉDÉ DE PRODUCTION DE 3-CYANO-3,5,5-TRIMÉTHYLCYCLOHEXANONE

(30) Priorität: 17.06.2011 DE 102011077681
(43) Veröffentlichungstag der Anmeldung: 23.04.2014
(73) Patentinhaber: Evonik Degussa GmbH, 45128 Essen (DE)
(72) Erfinder: SCHWARZ, Markus, 45721 Haltern am See (DE); MERKEL, Andreas, 45665 Recklinghausen (DE); NITZ, Jörg-Joachim, 45257 Essen (DE); GRUND, Gerda, 48653 Coesfeld (DE)
(86) Internationale Anmeldenummer: PCT/EP2012/060577
(87) Internationale Veröffentlichungsnummer: WO 2012/171830

(56) Entgegenhaltungen:
- EP-A2- 1 418 172
- WO-A1-2004/056753
- CN-A- 101 851 178
- GB-A- 887 413
- GB-A- 1 047 920
- US-A- 5 183 915
- DATABASE WPI Week 198234 Thomson Scientific, London, GB; AN 1982-71392E XP002682780, & JP 57 116038 A (NITTO CHEM IND CO LTD) 19. Juli 1982 (1982-07-19) in der Anmeldung erwähnt

## Beschreibung

Die vorliegende Erfindung bezieht sich auf die Herstellung von 3-Cyano-3,5,5-trimethylcyclohexanon (Isophoronnitril, kurz IPN) unter Verwendung eines Calciumalkoholates, im besonderen Calciumethanolat, als Katalysator.

Die Basen katalysierte Umsetzung von Blausäure (HCN) mit alpha, beta-ungesättigten cyclischen (oder acyclischen) Ketonen ist eine bekannte Reaktion (Hydrocyanation of Conjugated Carbonyl Compounds, Wataru Nagata and Mitsuru Yoshioka, publiziert online: 15. Juli 2005 DOI: 10.1002/0471264180.or025.03)

Die Herstellung von Isophoronnitril (IPN) als Vorstufe für die Synthese von Isophorondiamin wird großtechnisch durchgeführt.

Die Reaktion von Isophoron (IP) mit HCN zum IPN lässt sich mit folgender Reaktionsgleichung beschreiben:

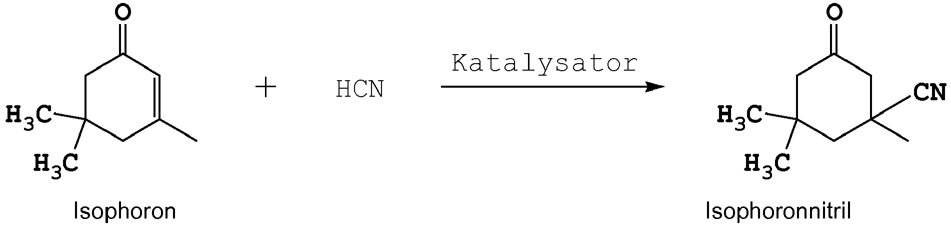

Die Katalysatoren für die Herstellung der beta-Cyanoketone wie z.B. IPN sind generell Basen, die freie Cyanid-Ionen (CN^{⊖}) für die 1,4-Additionsreaktion zur Verfügung stellen. Man unterscheidet dabei meistens zwischen anorganischen Salzen und organischen Verbindungen.

Blausäure ist eine schwache Säure mit einem pkₐ-Wert von 9,21 und addiert daher nicht von selbst an eine aktivierte Kohlenstoff-Kohlenstoff-Doppelbindung. Auf der anderen Seite ist CN^{⊖} (wie SH^{⊖} oder I^{⊖}) nukleophil genug, um an ein elektrophiles Kohlenstoffatom eines konjugierten Carbonyl-Systems zu addieren. Daher braucht man einen basischen Katalysator, um die Konzentration an CN^{⊖} Ionen zu erhöhen. Mögliche Katalysatoren sind daher Cyanid-haltige Salze (beispielsweise NaCN, KCN oder K₄Ni(CN₄), siehe

US 2 904 581), anorganische Basen (beispielsweise Kaliumcarbonat oder Natriumhydroxid) oder organische Basen (beispielsweise Triethylamin, Pyridin oder Piperidin).

Die Geschwindigkeit der Additionsreaktion ist proportional zur Konzentration an CN^{⊖} Ionen und die Addition ist in Bezug auf CN^{⊖} reversibel. Zudem steht die Reaktion zum Cyanhydrin in Konkurrenz zum Cyanoketon (1,2-Addition versus 1,4-Addition). Für viele Substrate ist die 1,2-Addition schneller als die 1,4-Addition.

Die Rückreaktion vom Cyanoketon (beispielsweise IPN) zum dementsprechenden Edukt (beispielsweise Isophoron) verläuft langsamer als die Hin-Reaktion. Die Rückreaktion der reversiblen Additionsreaktion ist mit steigender Temperatur begünstigt. Daher empfiehlt sich die Synthese bei möglichst niedrigen Temperaturen.

Das Cyanhydrin ist im basischen Medium nicht stabil, es bildet sich daher fast auschließlich ein Cyanoketon. Die Bildung des Cyanohydrin ist vernachlässigbar gering (bzw. reversibel, sodass CN^{⊖} Ionen gemäß 1,4-Addition abreagieren können).

Bei zu hoher CN^{⊖} Konzentration kann es zu einer stark exothermen spontanen Dimerisierung und/oder Polymerisation der Blausäure kommen. Polymerer Cyanwasserstoff, auch als Azulminsäure bezeichnet, entsteht durch anionische Polymerisation von HCN. Die Reaktion verläuft über dimere Blausäure (Iminoacetonitril). Gleichzeitig findet zwischen zwei gamma-ständigen Nitril-Gruppen ein Ringschluß statt, wodurch das kettenförmige Polymer stabilisiert wird. Diese Polymerisation gilt es in jedem Fall zu vermeiden, da dadurch die Selektivität der Isophoronnitril-Bildung negativ beeinflusst wird.

Die Umsetzung mit HCN kann in Lösung oder Lösemittelfrei durchgeführt werden.

### Stand der Technik

### a) Verfahren zur Herstellung von Isophoronnitril aus Isophoron und Blausäure:

JP06065183 Das alkalische Reaktionsgemisch wird mit einem inerten Lösemittel vermischt und in einem Dünnschichtverdampfer aufgearbeitet. Dabei werden der Katalysator, die Hochsieder und das Lösemittel abgetrennt. Das IPN wird dann in einer weiteren Kolonne aufgereinigt.

EP 0 554 786 (analog US 5 254 711) beschreibt ein Verfahren zur kontinuierlichen Herstellung von IPN durch basenkatalysierte Umsetzung von IP mit HCN in zwei getrennten Reaktionszonen. Die Herstellung von IPN erfolgt durch Umsetzung von IP mit Blausäure an einem alkalischen, cyanidionen bildenden Katalysator. Als geeignete Katalysatoren werden Alkalimetall- und Erdalkalimetall-alkoholate; ferner -hydroxyde und -cyanide; sowie quarternäre Ammoniumbasen genannt.

DE 102 59 708 (analog EP 1 581 481) beansprucht ein Verfahren zur Herstellung von IPN durch Umsetzung von IP mit HCN in Gegenwart basischer Katalysatoren - insbesondere Calciumoxid - und Zugabe von Sulfon- oder Carbonsäuren vor der weiteren destillativen Aufarbeitung. Durch Einsatz von Carbon- oder Sulfonsäuren kommt es nicht zur Bildung eines Niederschlages, das Rohprodukt kann sofort weiter aufgearbeitet werden.

Die bestehenden Katalysatoren und deren Verfahrenstechnischer Einsatz liefern als Stand der Technik Umsätze und Selektivitäten (98 % bezogen auf den Umsatz zu IPN) meist bei Temperaturen von > 170 °C unter Normaldruck.

Als mögliche Nebenreaktion tritt unter basischen Reaktionsbedingungen die Dimerisierung von Isophoron zum Diisophoron und / oder Polymerisationen ein. In der (Patent)Literatur ist die Selbstkondensation von Isophoron eingehend beschrieben:
1) J. A. Bertrand, D. Cheung, A. D. Hammerich, H. O. House, W. T. Reichle, D. Vanderveer, E. J. Zaiko, J. Org. Chem., 1977, 42, 9, 1600-1607.
2) G. Kabas, H. C. Rutz, The Alkali-Catalyzed Self-Condensation of Isophorone, Tetrahedron. 1966, 22, 1219-1226.
3) US 2 406 652, Ketols from Isophorones and Homologous Thereof.

Diese Dimerisierung gilt es bei der Herstellung von Isophoronnitril zu unterbinden, da diese Reaktion unter den bestehenden Synthesebedingungen nicht reversibel ist und somit

Isophoron verloren geht. Es bilden sich meist Hochsieder, die im Aufarbeitungsprozess abgetrennt werden müssen und somit die Selektivität (S) negativ beeinflussen.

### b) Katalysatoren zur Herstellung von IPN aus Isophoron und Blausäure, bestehend aus basischen Alkali- oder Erdalkalimetallverbindungen:

DE 10 85 871 mit dem Titel Verfahren zur Herstellung von alicyclischen Cyanketonen (analog GB 887 413 Carbocyclische Cyanoketone der ROHM & HAAS COMPANY) beschreibt die Herstellung von Cyanketonen durch Umsetzung von ungesättigten Ketonen mit Blausäure an einem alkalischen, cyanidionen bildenden Katalysator. Als geeignete Katalysatoren werden Alkalimetalle und ihre Carbonate; ferner Erdalkalimetalle und Alkali- und Erdalkalialkoholate, - oxyde, -hydroxyde, - peroxyde und -cyanide; tertiäre Amine und quarternäre Ammoniumbasen genannt. Der Katalysator ist in einer Menge von 0,1 bis 20 Gewichtsprozenten, bezogen auf das Gesamtgewicht der Reaktionsteilnehmer bezogen, erforderlich. Die Umsetzung erfolgt vorzugsweise bei Temperaturen von 150 bis 225 °C und bei Normaldruck. Die Herstellung von Isophoronniril wird als Beispiel genannt.

DE 12 40 521 Verfahren zur Herstellung von gamma-Ketocarbon-saeurenitrilen aus alpha,beta-ungesaettigten Ketonen: Es wird die Herstellung von IPN beschrieben, indem hohe Blausäurekonzentrationen und heterogene Katalysatoren (alkalische Katalysatoren auf Trägern, beispielsweise Tonscherben) ohne Lösemittel eingesetzt werden. Als basische Katalysatoren sind alle Substanzen geeignet, die unter den Reaktionsbedingungen in Gegenwart von Cyanwasserstoff Cyanidionen bilden. Dazu gehören z.B. Oxide, Hydroxide, Cyanide und Alkoholate der Alkali- und Erdalkalimetalle. Wesentlich ist, dass die Katalysatoren fest geträgert sind. Es wird bei Temperaturen zwischen 50 und 350 °C synthetisiert. Die Ausbeute beträgt 95,3 % Isohoronnitril, bezogen auf eingesetzte Blausäure, und 96,0 %, bezogen auf umgesetztes Isophoron.

DE 12 40 854 Verfahren zur Herstellung von 3-Cyan-3, 5, 5-trimethylcyclohexanon aus Isophoron und Blausäure: Beansprucht wird hier ein Verfahren zur Herstellung von IPN aus IP und HCN in Gegenwart alkalischer Katalysatoren bei 80 - 250 °C, indem 0,001 bis 0,1 Gew.-% Katalysator eingesetzt und ohne Lösemittel gearbeitet wird. Besonders geeignete Katalysatoren sind Alkaicyanide, -hydroxide und -alkoholate. Beispielhaft werden Ausbeuten von 96,2 % IPN, bezogen auf umgesetztes Isophoron, und 97,9 % IPN, bezogen auf eingesetzte Blausäure, erzielt.

EP 0 433 615 beinhaltet die Erzielung höherer Raum-Zeit-Ausbeute, einfache Durchführung und Vermeidung teurer Katalysatoren und Hilfsstoffe durch die Verwendung von Lithiumhydroxid (LiOH) als Katalysator. Es werden Ausbeuten von bis zu 96,1 % IPN, bezogen auf eingesetzten Cyanwasserstoff, erzielt.

EP 0 558 332 beansprucht die Verwendung von Lithiumcyanid als Katalysator, das zuvor aus LiOH hergestellt wurde. Die Konzentration an HCN wird so gewählt, dass kein freies LiOH gebildet wird. Die Menge an Nebenprodukten ist signifikant.

EP 1 418 172 beansprucht den Einsatz eines Calciumoxides mit einer Oberfläche von > 1,5 m²/g. Die Reaktion findet bevorzugt bei Temperaturen zwischen 150 - 170 °C statt.

### c) Katalysatoren zur Herstellung von Isophoronnitril aus Isophoron und Blausäure, bestehend aus Oniumverbindungen oder deren spezieller Verwendung:

JP 61033157 beansprucht Herstellung von IPN durch Reaktion von Blausäure und Isophoron in Anwesenheit von quaternären Ammonium- oder Phosphoniumhydroxiden als Katalysatoren.

EP 0 558 799 beansprucht bei der Umsetzung von Isopohoron mit HCN zur Herstellung von IPN den Einsatz eines Onium-Cyanids (beispielsweise Tetraalkylammonium-Cyanid oder Tributylsulfonium-Cyanid) als Katalysator. Die Reaktion wird zwischen 110 und 140 °C durchgeführt, liefert aber bis zu 2 % Diisophoron als Nebenkomponente.

EP 0 671 384 (analog US 5 516 928) beansprucht den Einsatz von quaterären Ammoniumsalzen mit Hydrogencarbonat bzw. Alkylcarbonat-Gegenionen. Reaktion von HCN mit Isophoron wird in einem Temperaturbereich zwischen 100 und 170 °C durchgeführt.

US 5 183 915 beschreibt die Herstellung von 3-Cyano-3,3,5-trialkylcyclohexanonen durch die Umsetzung des jeweiligen Enons mit HCN in der Abwesenheit von Wasser. Als Katalysator werden quarternäre Ammonium- oder Phosphoniumcyanide eingesetzt. Es entsteht ein deutlicher Anteil an Nebenprodukt (2,4 % Diisophoron).

EP 0 502 707 beansprucht den Einsatz von quaterären Ammonium- oder Phosphoniumsalzen und einem basischen Co-Katalysator (Kalium- oder Natriumcarbonat) bei der Synthese von IPN. Reaktion von HCN mit Isophoron wird in einem Temperaturbereich zwischen 90 und 140 °C durchgeführt.

In der Schrift US 5 011 968 wird die Herstellung von IPN durch Reaktion von Isophoron mit HCN und Tetramethylammoniumhydroxid als Katalysator und anschließender thermischer Zerstörung des Katalysators zu Trimethylamin und Entfernung mit dem Abgas beschrieben.

### d) Katalysatoren zur Herstellung von Isophoronnitril aus Isophoron und Blausäure, mittels Phasentransferkatalyse oder unter Verwendung eines zweiphasigen Reaktionssystems:

In JP 62164656 wird die Freisetzung von IPN aus dem entsprechenden Acetal des IPN durch Säure beschrieben.

EP 0 028 179 beschreibt die Herstellung von IPN aus Isophoron (organische Phase) und Cyaniden (wässrige Phase) in einem 2-Phasensystem mit einem, in katalytischen Mengen eingesetztem Phasenübertragungsmittel. Als Phasentransferkatalysatoren werden quaternäre Ammonium- und Phosphoniumsalze (beispielsweise Tetraethylammoniumbromid oder Dinatriumphosphat) eingesetzt, die in der organischen Phase löslich sind. Die Reaktionszeit beträgt beispielhaft 4 bis 5 Stunden bei 80 °C.

US 4 299 775 Verfahren zur Herstellung von 3-cyano-3,5,5-trimethylcyclohexanon beansprucht als Katalysator Natrium oder Kaliumcyanid in Gegenwart von Wasser und einem inerten Lösungsmittel sowie eines Onium-Phasentransferkatalysators.

EP 0 425 806 (DE 690 09 394T2) beschreibt die Umsetzung von Isophoron mit alkalischen Cyaniden in stöchiometrischen Mengen in homogener Lösung mit einem wässrigen und organischen Lösemittelgemisch, wobei gleichzeitig und allmählich mit anorganischen Säuren neutralisiert wird. Die Phasen entmischen sich während der Reaktion wieder und können auf diese Weise voneinander getrennt werden.

JP 04279558 beansprucht die Herstellung von IPN indem IP mit HCN in Gegenwart eines basischen Katalysators und geeigneter Mengen Wasser (0,1 - 5 %) bei 110 - 250 °C umgesetzt wird. Besonders geeignete Katalysatoren sind Alkaicyanide, -hydroxide und-alkoholate.

### e) Katalysatoren zur Herstellung von Isophoronnitril aus Isophoron und Blausäure, mittels besonderer organischer Basen:

EP 0 985 659 (analog DE 198 36 474) zeigt die Verwendung von 1,3-Dimethyl-imidazolium-4-carboxylat als Katalysator bei Temperaturen von 80 bis 220 °C, besonders bevorzugt 100 - 180 °C, und Atmosphärendruck.

JP 61033158 beschreibt die Herstellung von IPN durch Reaktion von Blausäure und Isophoron in Anwesenheit von Diazo-bicycloalkenen als Katalysatoren.

In JP 04253948 wird Isophoron zu IPN mit HCN in Gegenwart von Guanidin (0,01 - 0,5 Mol bezogen auf 1 Mol Isophoron) als Base umgesetzt.

### f) Katalysatoren zur Herstellung von Isophoronnitril aus Isophoron und Blausäure, mittels Einsatzes besonderer Lösemittel:

In JP 57116038 wird Isophoron zu IPN mit HCN in Gegenwart eines basischen Katalysators und einem Glykol (beispielsweise Etylenglykol) umgesetzt.

JP0 4164057 beschreibt die Herstellung von IPN durch äquimolare Umsetzung von IP mit HCN unter Basenkatalyse in 1,3-Dimethyl-2-imidazolidinon als Lösemittel.

JP 04112862 beinhaltet die Herstellung von IPN durch Reaktion von Isophoron mit HCN, alkalischen Katalysatoren und DMSO (Dimetylsulfoxid) und/oder DMF (Diemthylformamid als Lösemittel.

Es besteht Bedarf an basischen Katalysatoren für die Herstellung von beta-Cyanoketonen - IPN - die kostengünstig sind, hohen Umsatz und Selektivität ermöglichen und unter milden Reaktionsbedingungen aktiv sind. Zudem ist die Vermeidung von Abfallprodukten wünschenswert.

Die Aufgabe der vorliegenden Erfindung besteht also in der Bereitstellung eines Verfahrens und eines Katalysators für die Umsetzung von Isophoron mit Blausäure zum IPN bei niedriger Prozesstemperatur. Dabei war es besonders wichtig, einen Katalysator zu finden, der die Vorraussetzung zum Einsatz in einer großtechnischen Anlage erfüllt, insbesondere hohe Selektivität und hoher Umsatz.

Überraschender Weise wurde gefunden, dass Calciumalkoholate, im besondern Calciumethanolat, als heterogener Katalysator bei der Reaktion von Isophoron mit HCN zum Isophoronnitril (IPN) die gestellte Aufgabe löst.

Gegenstand der Erfindung ist ein Verfahren zur Herstellung von Isophoronnitril aus Isophoron und Blausäure, wobei die Reaktion in Gegenwart eines Calciumalkoholats als Katalysator in heterogener Phase durchgeführt wird, wobei die BET Oberfläche des Calciumalkoholats mindestens 5 m²/g beträgt und das Calciumalkoholat mit einer Schüttdichte von mindestens 250 g/l eingesetzt wird.
Bevorzugt wird als Katalysator Calciumethanolat eingesetzt.

Es wurde gefunden, dass der Katalysator, insbesondere das als Katalysator bevorzugt eingesetzte Calciumethanolat, die folgenden Vorteile aufweist und damit weitere Vorteile für das Verfahren gefunden wurden:
Bei Calciumethanolat handelt es sich um einen kostengünstigen heterogenen Katalysator, der bei Prozesstemperaturen kleiner 170 °C mit hohen Raum-Zeit-Ausbeuten betrieben werden kann. Mit einem solchen Katalysator lassen sich bereits unter Normaldruck bei einer Reaktionstemperatur von 90 °C Selektivitäten hinsichtlich des gewünschten Produkts Isophoronnitril (IPN) von bis zu größer 98 % erreichen und somit ist die Nebenproduktbildung (Diisophoron, polymere Blausäure und Hochsieder) sehr gering. Der Umsatz bezogen auf HCN beträgt dabei ebenfalls bis zu größer 98 %. Der erfindungsgemäße Katalysator kann sowohl in einem Festbett-, als auch in einem Slurry-Verfahren angewendet werden.

Desweiteren liefert die Verwendung der erfindungsgemäßen Katalysatoren bei niedriger Prozesstemperatur ein hohes Potential zur energetischen Prozeßoptimierung (Reduktion des Energiebedarfs) großtechnischer Verfahren.

Das als Katalysator eingesetzte Calciumalkoholat, bevorzugt das Calciumethanolat, weist die folgenden Eigenschaften auf:
Die BET Oberfläche des erfindungsgemäß eingesetzten Calciumalkoholats, bevorzugt Calciumethanolats, beträgt mindestens 5 m²/g, bevorzugt größer 8 m²/g, besonders bevorzugt größer 12 m²/g. Die Messung der BET Oberfläche ist nach dem statischvolumetrischen Mehrpunktverfahren gemäß DIN ISO 9277 (p/pₒ Bereich: 0,05 - 0,2) mittels einer Pulver förmigen Probe durchzuführen.

Weiterhin wird ein Calciumalkoholat, besonders bevorzugt ein Calciumethanolat eingesetzt, das eine Schüttdichte von mindestens 250 g/l aufweist. Unter der Schüttdichte ist das Verhältnis von Masse pro Volumen [Einheit: g/l] einer losen, nicht gestampften Schüttung zu verstehen. Besonders bevorzugt zeichnet sich das erfindungsgemäße Calciumalkoholat, bevorzugt Calciumethanolat, durch eine Schüttdichte im Bereich von 290 g/l bis 550 g/l aus.

Besonderes bevorzugt wird Calciumethanolat eingesetzt. Die BET Oberfläche, gemessen mittels einer Pulver förmigen Probe, des erfindungsgemäß eingesetzten Calciumethanolats beträgt mindestens 5 m²/g, bevorzugt größer 8 m²/g, besonders bevorzugt größer 12 m²/g. Ferner weist das Calciumethanolat eine Schüttdichte von mindestens 250 g/l, bevorzugt im Bereich von 290 g/l bis 550 g/l, auf. Außerdem weist das Calciumethanolat eine mittlere Partikelteilchengröße (d50-Wert) zwischen 10 - 200 µm. Diese hier genannten Bereiche können beliebig kombiniert werden.

Die dynamische Differenzkalorimetrie (differential scanning calorimetry, DSC, gemäß DIN EN ISO 11357-1 und DIN 53765) des erfindungsgemäß bevorzugten Calciumethanolat ergab endotherme Peak(s) im Bereich von 70 - 130 °C, und DSC-Wärmeströme von 0,35 bis 0,55 mW/mg.

Die DSC-Analyse ist im Temperaturbereich von -50 bis 300 °C und 10 K/min im Stickstoffstrom von 50 - 70 ml und einer Einwaage von 10 - 20 mg im Aluminiumpfännchen "pierced lid modus" z.B. mit einem DSC-Gerät NETZSCH DSC 204 F1 durchzuführen.

Die Partikelgrößenverteilung und die weiteren physikalischen Daten des erfindungsgemäß eingesetzten Calciumethanolat ist in der folgenden Tabelle 1 dargestellt. Der Medianwert der Partikelgrößenverteilung, auch d50-Wert genannt, ist als Maß für die mittlere Teilchengröße die wichtigste Kenngröße. 50 Volumen-Prozent der Probe sind feiner und die anderen 50% sind grober als der d50-Wert. Der Wert d10 ist analog definiert.

**Tab. 1: Physikalische Daten des erfindungsgemäß eingesetzten Calciumalkoholats**

| Katalysator | Schüttdichte des Pulvers [g/liter] | Partikelgrößen d10 [µm] | Partikelgrößen d50 [µm] | BET Oberfläche [m²/g] | DSC endotherm-Peak [°C] | DSC Wärmestrom [mW/mg] |
|---|---|---|---|---|---|---|
| 1 | 466 | 7 | 134 | 13 | 100 | 0,38 |
| 2 | 483 | 4 | 21 | 8 | 93 | 0,42 |
| 3 | 296 | 5 | 43 | 15 | 93 | 0,45 |
| 4 | 462 | 10 | 70 | 15 | 98 | 0,39 |

Der Katalysator kann als sowohl als Pulver, Pellets, Granulat, oder Extrudat vorliegen.

Generell kann die Herstellung von Isophoronnitril homogen oder heterogen Basen katalysiert durchgeführt werden. In der homogenen Katalyse liegen Katalysator und Reaktanden in der gleichen Phase vor. Typisch ist ein in einem Lösungsmittel gelöstes Metallsalz (z.B. Natriummethylat gelöst in Methanol), an dem die Reaktanden reagieren.

Im Folgenden werden die Vorteile des erfindungsgemäßen Verfahrens in heterogener Phase beschrieben. In der erfindungsgemäß eingesetzten heterogenen Katalyse liegen Katalysator und Reaktanden in unterschiedlichen Phasen vor. Der feste Katalysator ist der in flüssiger Phase den vorliegenden Reaktanden ausgesetzt, also zwischen dem Katalysator und dem Reaktionsgemisch ist eine Phasengrenze vorhanden. Der Katalysator ist ein poröser Feststoff, während die Reaktanden gasförmig und/oder flüssig sind. Bei heterogen katalysierten Prozessen spielen sich die entscheidenden Vorgänge an der Oberfläche des Festkörpers ab.

Die erfindungsgemäß eingesetzte heterogene Katalyse birgt im Gegensatz zur klassischen homogenen Katalyse den für die industrielle Umsetzung unschätzbaren Vorteil einer einfachen Katalysatorabtrennung, bedingt durch die Existenz einer zweiten Phase in sich. Damit ist die heterogene Katalyse ein oberflächenkontrollierter Prozess, was bedeutet, dass physikalische Stofftransportprozesse, wie der Stofftransport in porösen Katalysatoren, die erzielbaren Umsätze und Selektivitäten maßgeblich beeinflussen können.

Die Beständigkeit eines Katalysators gegenüber chemischen, thermischen oder mechanischen Einflüssen bestimmt dessen Lebenszeit in Reaktoren (Standzeit des Katalysators in einem Festbettreaktor). Durch Zersetzung, Verkokung und Vergiftung des Katalysators, kann dessen Stabilität stark beeinflusst werden. Ob ein Katalysator daher für einen industriellen Prozess geeignet ist, hängt von seiner Aktivität, Selektivität und Stabilität ab.

Mit dem erfindungsgemäß eingesetzten Calciumalkoholat, bevorzugt Calciumethanolat, wurden überraschender Weise Katalysatoren gefunden, die diese Anforderungen erfüllen.

Mit dem erfindungsgemäß verwendeten Katalysator sind besonders kurze Verweilzeiten des Reaktionsgemisches im Reaktor bei guten Ausbeuten, Selektivitäten und hohen Raum-Zeit-Ausbeuten erzielbar. Je nach den gewählten Reaktionsbedingungen betragen die Verweilzeiten wenige Minuten bis wenige Stunden.

Die Umsetzung kann in Gegenwart oder Abwesenheit von inerten Lösungsmitteln vorgenommen werden. Besonders bevorzugt verwendet man Isophoron im molaren Überschuss, bezogen auf HCN, und setzt kein externes Lösungsmittel zu.

Aufgrund der notwendigen, niedrigen HCN-Konzentrationen ist das Vorlegen von HCN mit anschließender Zugabe von Isophoron nicht sinnvoll. Es wird also immer zumindest eine Teilmenge des Isophorons vorgelegt und das HCN bei einer Temperatur zugefügt, bei der die Addition an Isophoron mit der gewünschten Geschwindigkeit eintritt. Dabei muss auch der Katalysator anwesend sein. HCN darf auch nicht zu langsam zugegeben werden, da hierbei, insbesondere bei hohen Reaktionstemperaturen, Isophoronnitril unter Neubildung von HCN und Isophoron zerfällt.

Da - wie bereits erwähnt - das HCN nicht derart zugegeben werden darf, dass eine zur Polymerisation ausreichende Menge davon (die nicht zu Cyanidionen umgesetzt und somit nicht direkt an das Isophoron addiert) in dem Reaktionsgemisch vorliegt, darf weiterhin die Geschwindigkeit der Zudosierung von HCN zu Isophoron auch nicht zu hoch gewählt werden.

Generell wird das erfindungsgemäße Verfahren derart durchgeführt, dass ein Überschuss an Isophoron eingesetzt wird, da so eine höhere Selektivität zu Isophoronnitril erzielt wird. Im Allgemeinen beträgt das molare Verhältnis Isophoron/HCN >1:1, in der Regel 19 : 1 bis 1,2 : 1, vorzugsweise 3 : 1 bis 1,5 : 1.

Die Gesamtmenge des Isophorons kann vorgelegt und auf die gewünschte Reaktionstemperatur gebracht werden, bevor in Anwesenheit des Katalysators das HCN zugegeben wird. Es hat sich als günstig erwiesen, einen Teil des Isophorons vorzulegen und nach Erwärmen auf Reaktionstemperatur ein Gemisch aus Isophoron und HCN in einem geeigneten Verhältnis in Anwesenheit des Katalysators zuzugeben.

Der Katalysator kann bereits während des Aufwärmens des Isophorons anwesend sein. Im Unterschied zu den üblichen, nach dem Stand der Technik verwendeten Katalysatoren, insbesondere Alkalisalzen, tritt dabei keine oder nur eine untergeordnete Polymerisation des Isophorons ein.

Innerhalb der vorstehend dargelegten Reaktionsparameter wird das HCN bzw. das Isophoron/HCN-Gemisch so zudosiert, dass eine ausreichend niedrige Stationärkonzentration von HCN resultiert und eine hohe Selektivität sowie ein hoher Umsatz zu Isophoronnitril erzielt werden. Dabei darf nur eine geringe Polymerisation des HCN auftreten, da hierdurch der Umsatz und die Selektivität negativ beeinflusst würden. Die Selektivität hinsichtlich IPN sollte > 95 % bevorzugt > 98 %, besonders bevorzugt > 99 %, liegen.

Vorzugsweise werden die stationären Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen angepasst, bis die Werte in dem gewünschten Rahmen liegen. Die Bestimmung der genannten Cyanidionen-Konzentrationen erfolgt vorzugsweise durch Titration.

Die Aufarbeitung des Reaktionsgemisches nach beendeter Reaktion geschieht auf übliche, dem Fachmann bekannte Weise. Vorzugsweise wird überschüssiges Isophoron durch Destillation abgetrennt und vorteilhafterweise erneut eingesetzt. Danach wird das gebildete Isophoronnitril, vorzugsweise ebenfalls destillativ, von Hochsiedern und eingesetztem Katalysator getrennt.

Bei den, nach dem Stand der Technik verwendeten basischen Katalysatoren, tritt eine teilweise Polymerisation des HCN und Nebenreaktionen des Isophorons (Dimerisierung und Polymerisationen) ein. Es ist bevorzugt, diese Reaktion durch eine geringe freie Konzentration an Cyanid-Ionen zu unterdrücken. Dies impliziert einen hohen Umsatz an HCN. Bei dem erfindungsgemäßen Verfahren wird ein Umsatz von mindestens 70 % erreicht. Je nach gewählter Katalysatorkonzentration lassen sich aber bereits bei einer Reaktionstemperatur von 90 °C Umsätze von > 95 %, bevorzugt > 98 %, besonders bevorzugt > 99 % erzielen.

Ein weiterer Vorteil des erfindungsgemäßen Verfahrens liegt in seiner ökologischen und ökonomischen Ausführung. In bevorzugter Weise wird die katalytische Reaktion bei einer Reaktionstemperatur im Bereich von 80 bis 180 °C, besonders bevorzugt 80 bis 130 °C und ganz besonders bevorzugt 80 bis 100 °C ausgeführt.

Die Reaktion wird bei Drücken von 0,01 bis 10 bar, bevorzugt 1 bis 5 bar, besonders bevorzugt bei Normaldruck (Atmosphärendruck) durchgeführt.

Das erfindungsgemäße Verfahren besitzt daher den Vorteil, dass es kostengünstiger und wirtschaftlicher durchgeführt werden kann und auch aufgrund der kürzeren Reaktionsdauer einen größeren Umsatz pro Zeiteinheit bietet.

Die benötigte Reaktionszeit für die katalytische Reaktion beträgt vorzugsweise 5 Minuten bis 180 Minuten, besonders bevorzugt 5 Minuten bis 90 Minuten.

Diese Reaktionszeiten sind im Vergleich zum Stand der Technik erheblich niedriger. Aus den Beispielen der dort genannten Druckschriften geht hervor, dass sowohl die Reaktionszeiten, als auch die Reaktionstemperaturen deutlich länger bzw. höher liegen als bei dem erfindungsgemäßen Verfahren und somit ein deutlich erhöhter Energieaufwand nötig ist, um die Reaktion durchzuführen.

Das erfindungsgemäße Verfahren kann kontinuierlich, diskontinuierlich oder semikontinuierlich durchgeführt werden.

In bevorzugter Ausführung wird die Reaktionsdurchführung in einem Rührkessel, einer Rührkesselkaskade, einem Strömungsrohr, einem oder mehreren Festbettreaktoren oder einer Kolonne durchgeführt. Die katalytische Reaktion wird an einem heterogenen Katalysator durchgeführt.

Nach der Anordnung der festen Phase im Reaktionsapparat unterscheidet man prinzipiell zwei Klassen von Reaktoren für heterogen-katalytische Reaktionsprozesse und können bei dem erfindungsgemäßen Verfahren zum Einsatz kommen:
1. Die feste Phase ist unbeweglich im Reaktor angeordnet (Festbett-Verfahren, z. B. Festbettreaktor),
2. Der Katalysator ist mobil (Slurry-Verfahren, z. B. Suspensionsreaktor).

Das erfindungsgemäße Verfahren kann mittels einer dreiphasigen Reaktion durchgeführt. Bei dreiphasigen Reaktionen, dass heißt in Gas-Flüssig-Fest-Prozessen, wirkt der Feststoff als Katalysator. Die Reaktanten können sowohl in der Gas-, als auch in der Flüssigphase enthalten sein. Die fluiden Phasen können auch teilweise Reaktionsprodukt enthalten.

Die heterogen katalysierte Reaktion in (halb)diskontinuierlichen Suspensions-Reaktoren gemäß 2. ist im Rahmen der Erfindung durchführbar.

Besonders bevorzugt wird erfindungsgemäß ein Festbettreaktor gemäß 1. eingesetzt.

Erfindungsgemäß wird das Verfahren bevorzugt in einem kontinuierlich betriebenen Festbettreaktor durchgeführt. Dabei wird eine Filtration des Katalysators gemäß der Verfahrensvariante 2. umgangen und es können sehr hohe Raumzeitausbeuten erreicht werden.

Ganz besonders bevorzugt wird das erfindungsgemäße Verfahren zweiphasig durchgeführt, bevorzugt mittels eines kontinuierlich betriebenen Festbettreaktors mit Vorsättiger. Dort wird die Flüssigkeit mit Gas (auch HCN) gesättigt und anschließend nur die gesättigte Flüssigkeit in einen Festbettreaktor geleitet. Man hat somit ein zwei Phasen System (flüssig/fest). Besonders bevorzugt wird hierbei Isophoron im molaren Überschuss, bezogen auf HCN, verwendet, wobei Isophoron somit das Lösemittel darstellt und es wird kein externes Lösungsmittel eingesetzt.

Gegenstand der Erfindung ist auch die Verwendung von partikelförmigem Calciumethanolat mit einer BET Oberfläche, gemessen mittels einer Pulver förmigen Probe, von mindestens 5 m²/g, bevorzugt größer 8 m²/g, besonders bevorzugt größer 12 m²/g, und einer Schüttdichte von mindestens 250 g/l, bevorzugt im Bereich von 290 g/l bis 550 g/l, wobei die Bereiche beliebig kombiniert werden können, als Katalysator zur Herstellung von Isophoronnitril aus Isophoron und Blausäure, wobei die Reaktion in heterogener Phase, bevorzugt zweiphasig, bevorzugt in einem Festbettreaktor; durchgeführt wird. Alternativ kann die Reaktion in einem Suspensionsreaktor durchgeführt werden..

### Beispiele und Vergleichsbeispiele

Die Versuche zur Bildung von Isophoronnitril aus Isophoron und Blausäure mittels verschiedener homogener und heterogener Katalysatoren wurden isotherm und diskontinuierlich bzw. halbkontinuierlich in einer Rührkesselapparatur mit Tropftrichter und Rückflusskühler durchgeführt. Die Durchführung eines erfolgte derart, dass die Hauptmenge Isophoron (711 g) und eine Teilmenge der Blausäure (7 g (stabilisiert mit 0,5 - 1,0 % Phosphorsäure)) im Reaktor vorgelegt und auf die gewünschte Reaktionstemperatur gebracht wurden. Die restliche Blausäure (61 g) wurde in einem Gemisch mit Isophoron (118 g) über einem Tropftrichter zugegeben. Der Zeitpunkt t = 0 wurde durch die Zugabe des Katalysators festgelegt. Kurz vor der Zugabe des Katalysators wurde die Zusammensetzung des Reaktionsgemisches durch eine Probenahme und Gaschromatographische Analyse bestimmt, um mögliche Änderungen während der Aufheizphase zu berücksichtigen. Nach Zugabe des Katalysators wurde die Restmenge Blausäure/Isophoron kontinuierlich zugetropft. Die Zudosierung der Blausäure darf nicht zu schnell erfolgen, da es sonst durch die gebildeten Cyanid-Ionen zu einer Polymerisation der Blausäure kommt. Es entsteht dabei ein brauner polymerer Feststoff, der abgetrennt werden muss. Zu verschiedenen Zeitpunkten wurden Proben genommen und analysiert (Bestimmung des Cyanidgehaltes durch Titration und GC-Bestimmung). Nach abgeschlossener Reaktion wird noch 30 Minuten nachgerührt.

### Gaschromatographische Bestimmung der Reaktionsprodukte:

Gerät: HP3 / Agilent GC 6890
Tennsäule: HP-5 Agilent (19091J-433) 30m x 250µm x 0.25µm nominal
Const. Flow: 0,9 ml/min
Injektor: Temperatur: 200 °C
Total Flow: 84,6 ml/min
Split Flow: 81,1 ml/min (1:94,8)
Detektor: Temperatur: 250 °C
H₂ Flow: 40 ml/min
Air Flow: 450 ml/min
Make-up Flow (N₂): 45 ml/min
Ofen: Temperaturprogramm: 90°C/3min mit 5°C/min auf 150°C
mit 10 °C/min auf 300 °C/ 29min
Probenvorbreitung: 1000 mg Probe + 70 mg C-12 als ISTD in 10 ml Toluol
Injektionsvolumen: 1,0 µL
Auswertung : ISTD %

**Tabelle 2: Vergleichsbeispiele mit homogenen Katalysatoren**

| **Verbindungsklasse** | **Alkoholat** | **Alkoholat** | **Alkoholat** | **Alkoholat** | **Alkoholat** |
|---|---|---|---|---|---|
| Homogener Katalysator | LiOMe | KOMe | NaOMe | NaOMe | NaOMe |
| Katalysatormenge [g] | 6,0 | 4,4 | 3,4 | 3,4 | 3,4 |
| Temperatur [°C] | 150 | 150 | 150 | 120 | 90 |
| HCN-Umsatz [%] | 98 | 93 | 98 | 82 | 54 |
| Ausbeute (IPN in %) | 45 | 40 | 46 | 32 | 20 |
| Selektivität (IPN in %) | 95 | 98 | 99 | 99 | 98 |
| Reaktionszeit [min] | 345 | 266 | 305 | 215 | 216 |

Fazit: verschiedene Alkoholate zeigen als homogene Katalysatoren bei 150 °C hinsichtlich der Ausbeute und der Selektivität eine gute Performance, insbesondere NaOMe zeigt als homogener Katalysator eine sehr gute Performance. Mit sinkender Temperatur nimmt der Umsatz jedoch deutlich ab.

**Tabelle 3: Vergleichsbeispiele mit heterogenen Katalysatoren**

| **Verbindungsklasse** | **Oxid** | **Oxid** | **Carbonat** | **Carbonat** | **Carbonat** | **Carbonat** |
|---|---|---|---|---|---|---|
| Heterogener Katalysator | MgO (> 98 %) | CaO (> 95 %) | Li₂CO₃ (> 99 %) | Na₂CO₃ (> 99 %) | K₂CO₃ (> 99 %) | CaCO₃ (> 99 %) |
| Hersteller / Lieferant | Fluka | FelsWerke | Aldrich | Riedel de Haen | Merck | Aldrich |
| Katalysatormenge [g] | 10,0 | 10,0 | 10,0 | 10,0 | 10,0 | 10,0 |
| Temperatur [°C] | 170 | 170 | 170 | 170 | 170 | 170 |
| HCN-Umsatz [%] | 24 | 96 | 17 | 82 | 95 | <1 |
| Ausbeute (IPN in %) | 1 | 43 | 0,6 | 42 | 42 | < 1 |
| Selektivität (IPN in % | 67 | 99 | 67 | 92 | 99 | 6 |
| Reaktionszeit [min] | 248 | 145 | 103 | 202 | 120 | 102 |

Fazit: Verschiedenste basische heterogene Katalysatoren zeigen bei hohen Temperaturen von T = 170 °C teilweise eine akzeptable Performance hinsichtlich Ausbeute und Selektivität. Die Reaktionszeiten und die Reaktionstemperaturen sind jedoch relativ hoch.

**Tabelle 4: Beispiele gemäß der Erfindung**

| Der verwendete partikelförmige Ca(OEt)₂ Katalysator weist die folgenden Eigenschaften auf: | | | | | | |
|---|---|---|---|---|---|---|
| BET: 17m²/g | | | | | | |
| Partikelgröße: 20 µm (d50-Wert) | | | | | | |
| Schüttdichte: 460 g/L | | | | | | |
| **Verbindungsklasse** | **Alkoholat** | **Alkoholat** | **Alkoholat** | **Alkoholat** | **Alkoholat** | **Alkoholat** |
| Heterogener Katalysator | Ca(OEt)₂ | Ca(OEt)₂ | Ca(OEt)₂ | Ca(OEt)₂ | Ca(OEt)₂ | Ca(OEt)₂ |
| Katalysatormenge [g] | 2,05 | 2,05 | 2,05 | 2,05 | 4,10 | 8,20 |
| Temperatur [°C] | 150 | 130 | 110 | 90 | 90 | 90 |
| HCN-Umsatz [%] | 97 | 92 | 83 | 75 | 96 | >99 |
| Ausbeute (IPN in %) | 44 | 41 | 37 | 33 | 44 | 45 |
| S (IPN bezogen auf IP) | >99 | >99 | >99 | >99 | 99 | 95 |
| Reaktionszeit [min] | 76 | 76 | 76 | 76 | 71 | 76 |

Fazit: Eine herausragende Performance hinsichtlich Umsatz, Selektivität und niedriger Reaktionszeit wird bei dem erfindungsgemäßen heterogenen Katalysator Ca(OEt)₂ festgestellt. Selbst bei 90 °C zeigt das erfindungsgemäße Ca(OEt)₂ noch eine exzellente IPN Ausbeute bei nahezu gleichbleibend hoher Selektivität. Je nach eingesetzter Katalysatormenge wird ein HCN-Umsatz von bis zu größer 99 % erzielt.

## Patentansprüche

1. Verfahren zur Herstellung von Isophoronnitril aus Isophoron und Blausäure, wobei die Reaktion in Gegenwart eines Calciumalkoholats als Katalysator in heterogener Phase durchgeführt wird,
**dadurch gekennzeichnet,**
**dass** die BET Oberfläche des Calciumalkoholats, mindestens 5 m²/g beträgt
und
**dadurch gekennzeichnet,**
**dass** Calciumalkoholat mit einer Schüttdichte von mindestens 250 g/l eingesetzt wird.

2. Verfahren zur Herstellung von Isophoronnitril nach mindestens einem der vorherigen Ansprüche, **dadurch gekennzeichnet, dass** die Reaktion in Gegenwart von Calciumethanolat als Katalysator in heterogener Phase durchgeführt wird.

3. Verfahren zur Herstellung von Isophoronnitril nach mindestens einem der vorherigen Ansprüche,
**dadurch gekennzeichnet,**
**dass** die BET Oberfläche, gemessen mittels einer Pulver förmigen Probe, des Calciumethanolats mindestens 5 m²/g beträgt, und wobei das Calciumethanolat eine Schüttdichte von mindestens 250 g/l aufweist.

4. Verfahren zur Herstellung von Isophoronnitril nach mindestens einem der vorherigen Ansprüche,
**dadurch gekennzeichnet,**
**dass** das Calciumethanolat im DSC endotherme Peak(s) im Bereich von 70 -130°C und DSC-Wärmeströme von 0,35 bis 0,55 mW/mg aufweist.

5. Verfahren zur Herstellung von Isophoronnitril nach mindestens einem der vorherigen Ansprüche,
**dadurch gekennzeichnet,**
**dass** das Calciumethanolat eine mittlere Partikelteilchengröße (d50-Wert) zwischen 10-200 µm aufweist.

6. Verfahren zur Herstellung von Isophoronnitril nach mindestens einem der vorherigen Ansprüche,
**dadurch gekennzeichnet,**
**dass** Calciumethanolat eingesetzt wird, mit einer BET Oberfläche von mindestens 8 m²/g, und einer Schüttdichte im Bereich von 290 g/l bis 550 g/l.

7. Verfahren zur Herstellung von Isophoronnitril nach mindestens einem der vorherigen Ansprüche,
**dadurch gekennzeichnet,**
**dass** Calciumethanolat eingesetzt wird, mit einer BET Oberfläche von größer 12 m²/g, und einer Schüttdichte im Bereich von 290 g/l bis 550 g/l.

8. Verfahren zur Herstellung von Isophoronnitril nach mindestens einem der vorherigen Ansprüche,
**dadurch gekennzeichnet,**
**dass** Katalysator als Pulver, Pellets, Granulat, oder Extrudat vorliegt.

9. Verfahren zur Herstellung von Isophoronnitril nach mindestens einem der vorherigen Ansprüche,
**dadurch gekennzeichnet,**
**dass** in Gegenwart oder Abwesenheit von inerten Lösungsmitteln gearbeitet wird.

10. Verfahren zur Herstellung von Isophoronnitril nach mindestens einem der vorherigen Ansprüche,
**dadurch gekennzeichnet,**
**dass** Isophoron im molaren Überschuss, bezogen auf HCN, eingesetzt wird und kein externes Lösungsmittel zugegeben wird.

11. Verfahren zur Herstellung von Isophoronnitril nach mindestens einem der vorherigen Ansprüche,
**dadurch gekennzeichnet,**
**dass** das molare Verhältnis Isophoron/HCN > 1 : 1 beträgt.

12. Verfahren zur Herstellung von Isophoronnitril nach mindestens einem der vorherigen Ansprüche,
**dadurch gekennzeichnet,**
**dass** das molare Verhältnis Isophoron/HCN 19 : 1 bis 1,2 : 1 beträgt.

13. Verfahren zur Herstellung von Isophoronnitril nach mindestens einem der vorherigen Ansprüche,
**dadurch gekennzeichnet,**
**dass** ein Teil des Isophorons vorgelegt wird und nach Erwärmen auf Reaktionstemperatur ein Gemisch aus Isophoron und HCN in einem molare Verhältnis Isophoron/HCN > 1 : 1, in Anwesenheit des Katalysators zuzugeben wird.

14. Verfahren zur Herstellung von Isophoronnitril nach mindestens einem der vorherigen Ansprüche,
**dadurch gekennzeichnet,**
**dass** die Reaktion bei einer Reaktionstemperatur im Bereich von 80 bis 180 °C durchgeführt wird.

15. Verfahren zur Herstellung von Isophoronnitril nach mindestens einem der vorherigen Ansprüche,
**dadurch gekennzeichnet,**
**dass** die Reaktion bei Drücken von 0,01 bis 10 bar durchgeführt wird.

16. Verfahren zur Herstellung von Isophoronnitril nach mindestens einem der vorherigen Ansprüche,
**dadurch gekennzeichnet,**
**dass** wobei Reaktionszeit 5 Minuten bis 180 Minuten beträgt.

17. Verfahren zur Herstellung von Isophoronnitril nach mindestens einem der vorherigen Ansprüche,
**dadurch gekennzeichnet,**
**dass** zweiphasig durchgeführt wird.

18. Verfahren zur Herstellung von Isophoronnitril nach mindestens einem der vorherigen Ansprüche,
**dadurch gekennzeichnet,**
**dass** das Verfahren in einem kontinuierlich betriebenen Festbettreaktor durchgeführt wird.

19. Verfahren zur Herstellung von Isophoronnitril nach mindestens einem der vorherigen Ansprüche,
**dadurch gekennzeichnet,**
**dass** das Verfahren zweiphasig mittels einem kontinuierlich betriebenen Festbettreaktor mit Vorsättiger durchgeführt wird.

20. Verfahren zur Herstellung von Isophoronnitril nach mindestens einem der vorherigen Ansprüche 1 - 17,
**dadurch gekennzeichnet,**
**dass** ein Slurry Verfahren (Pulver in Lösung) eingesetzt wird.

21. Verwendung von partikelförmigem Calciumethanolat mit einer BET Oberfläche, gemessen mittels einer pulverförmigen Probe, von mindestens 5 m²/g und einer Schüttdichte von mindestens 250 g/l als Katalysator zur Herstellung von Isophoronnitril aus Isophoron und Blausäure, wobei die Reaktion in heterogener Phase durchgeführt wird.

## Claims

1. Method for preparing isophorone nitrile from isophorone and hydrocyanic acid, wherein the reaction is carried out in a heterogeneous phase in the presence of a calcium alkoxide as catalyst, **characterized in that**
the BET surface area of the calcium alkoxide is at least 5 m²/g
and
**characterized in that**
calcium alkoxide is used with an apparent density of at least 250 g/l.

2. Method for preparing isophorone nitrile according to at least one of the preceding claims, **characterized in that** the reaction is carried out in a heterogeneous phase in the presence of calcium ethoxide as catalyst.

3. Method for preparing isophorone nitrile according to at least one of the preceding claims,
**characterized in that**
the BET surface area of the calcium ethoxide, measured using a pulverulent sample, is at least 5 m²/g, and wherein the calcium ethoxide has an apparent density of at least 250 g/l.

4. Method for preparing isophorone nitrile according to at least one of the preceding claims,
**characterized in that**
the calcium ethoxide has endothermic peak(s) in the range of 70 - 130°C and DSC heat flows of 0.35 to 0.55 mW/mg in DSC.

5. Method for preparing isophorone nitrile according to at least one of the preceding claims,
**characterized in that**
the calcium ethoxide has an average particle size (d50 value) between 10-200 µm.

6. Method for preparing isophorone nitrile according to at least one of the preceding claims,
**characterized in that**
calcium ethoxide is used having a BET surface area of at least 8 m²/g, and an apparent density in the range of 290 g/l to 550 g/l.

7. Method for preparing isophorone nitrile according to at least one of the preceding claims,
**characterized in that**
calcium ethoxide is used having a BET surface area of greater than 12 m²/g, and an apparent density in the range of 290 g/l to 550 g/l.

8. Method for preparing isophorone nitrile according to at least one of the preceding claims,
**characterized in that**
catalyst is present as powder, pellets, granulate, or extrudate.

9. Method for preparing isophorone nitrile according to at least one of the preceding claims,
**characterized in that**
said method is carried out in the presence or absence of inert solvents.

10. Method for preparing isophorone nitrile according to at least one of the preceding claims,
**characterized in that**
isophorone is used in molar excess, based on HCN, and no external solvent is added.

11. Method for preparing isophorone nitrile according to at least one of the preceding claims,
**characterized in that**
the molar ratio of isophorone/HCN is > 1 : 1.

12. Method for preparing isophorone nitrile according to at least one of the preceding claims,
**characterized in that**
the molar ratio of isophorone/HCN is 19 : 1 to 1.2 : 1.

13. Method for preparing isophorone nitrile according to at least one of the preceding claims,
**characterized in that**
a portion of the isophorone is charged and, after heating to reaction temperature, a mixture of isophorone and HCN is added in a molar ratio of isophorone/HCN > 1 : 1 in the presence of the catalyst.

14. Method for preparing isophorone nitrile according to at least one of the preceding claims,
**characterized in that**
the reaction is carried out at a reaction temperature in the range of 80 to 180°C.

15. Method for preparing isophorone nitrile according to at least one of the preceding claims,
**characterized in that**
the reaction is carried out at pressures of 0.01 to 10 bar.

16. Method for preparing isophorone nitrile according to at least one of the preceding claims,
**characterized in that**
the reaction time is 5 minutes to 180 minutes.

17. Method for preparing isophorone nitrile according to at least one of the preceding claims,
**characterized in that**
said method is carried out biphasically.

18. Method for preparing isophorone nitrile according to at least one of the preceding claims,
**characterized in that**
the method is carried out in a fixed-bed reactor operated in continuous mode.

19. Method for preparing isophorone nitrile according to at least one of the preceding claims,
**characterized in that**
the method is carried out biphasically by means of a fixed-bed reactor operated in continuous mode with presaturator.

20. Method for preparing isophorone nitrile according to at least one of the preceding Claims 1 - 17,
**characterized in that**
a slurry method (powder in solution) is used.

21. Use of particulate calcium ethoxide having a BET surface area, measured using a pulverulent sample, of at least 5 m²/g and an apparent density of at least 250 g/l as catalyst for preparing isophorone nitrile from isophorone and hydrocyanic acid, wherein the reaction is carried out in a heterogeneous phase.

## Revendications

1. Procédé pour la préparation d'isophoronenitrile à partir d'isophorone et d'acide cyanhydrique, la réaction étant effectuée en phase hétérogène en présence d'un alcoolate de calcium comme catalyseur, **caractérisé en ce que** la surface BET de l'alcoolate de calcium vaut au moins 5 m²/g et
**caractérisé en ce qu'**on utilise un alcoolate de calcium présentant une densité apparente d'au moins 250 g/l.

2. Procédé pour la préparation d'isophoronenitrile selon au moins l'une quelconque des revendications précédentes, **caractérisé en ce que** la réaction est effectuée en phase hétérogène en présence d'éthanolate de calcium comme catalyseur.

3. Procédé pour la préparation d'isophoronenitrile selon au moins l'une quelconque des revendications précédentes, **caractérisé en ce que** la surface BET, mesurée au moyen d'un échantillon sous forme de poudre, de l'éthanolate de calcium est d'au moins 5 m²/g, l'éthanolate de calcium présentant une densité apparente d'au moins 250 g/l.

4. Procédé pour la préparation d'isophoronenitrile selon au moins l'une quelconque des revendications précédentes, **caractérisé en ce que** l'éthanolate de calcium présente, dans la DSC (analyse calorimétrique différentielle), un/des pics endothermiques dans la plage de 70-130°C et des flux thermiques de DSC de 0,35 à 0,55 mW/mg.

5. Procédé pour la préparation d'isophoronenitrile selon au moins l'une quelconque des revendications précédentes, **caractérisé en ce que** l'éthanolate de calcium présente une grosseur moyenne de particule (valeur d50) entre 10-200 µm.

6. Procédé pour la préparation d'isophoronenitrile selon au moins l'une quelconque des revendications précédentes, **caractérisé en ce qu'**on utilise de l'éthanolate de calcium présentant une surface BET d'au moins 8 m²/g et une densité apparente dans la plage de 290 g/l à 550 g/l.

7. Procédé pour la préparation d'isophoronenitrile selon au moins l'une quelconque des revendications précédentes, **caractérisé en ce qu'**on utilise de l'éthanolate de calcium présentant une surface BET supérieure à 12 m²/g et une densité apparente dans la plage de 290 g/l à 550 g/l.

8. Procédé pour la préparation d'isophoronenitrile selon au moins l'une quelconque des revendications précédentes, **caractérisé en ce que** le catalyseur se trouve sous forme de poudre, de pellets, de granulat ou de produit extrudé.

9. Procédé pour la préparation d'isophoronenitrile selon au moins l'une quelconque des revendications précédentes, **caractérisé en ce qu'**on travaille en présence ou en l'absence de solvants inertes.

10. Procédé pour la préparation d'isophoronenitrile selon au moins l'une quelconque des revendications précédentes, **caractérisé en ce que** l'isophorone est utilisé en excès molaire, par rapport au HCN, et on n'ajoute pas de solvant externe.

11. Procédé pour la préparation d'isophoronenitrile selon au moins l'une quelconque des revendications précédentes, **caractérisé en ce que** le rapport molaire isophorone/HCN est > 1:1.

12. Procédé pour la préparation d'isophoronenitrile selon au moins l'une quelconque des revendications précédentes, **caractérisé en ce que** le rapport molaire isophorone/HCN vaut 19:1 à 1,2:1.

13. Procédé pour la préparation d'isophoronenitrile selon au moins l'une quelconque des revendications précédentes, **caractérisé en ce qu'**une partie de l'isophorone est disposée au préalable et après chauffage à la température de réaction, un mélange d'isophorone et de HCN dans un rapport molaire isophorone/HCN > 1:1 est ajouté en présence du catalyseur.

14. Procédé pour la préparation d'isophoronenitrile selon au moins l'une quelconque des revendications précédentes, **caractérisé en ce que** la réaction est effectuée à une température de réaction dans la plage de 80 à 180°C.

15. Procédé pour la préparation d'isophoronenitrile selon au moins l'une quelconque des revendications précédentes, **caractérisé en ce que** la réaction est effectuée à des pressions de 0,01 à 10 bars.

16. Procédé pour la préparation d'isophoronenitrile selon au moins l'une quelconque des revendications précédentes, **caractérisé en ce que** le temps de réaction est de 5 minutes à 180 minutes.

17. Procédé pour la préparation d'isophoronenitrile selon au moins l'une quelconque des revendications précédentes, **caractérisé en ce qu'**il est effectué dans deux phases.

18. Procédé pour la préparation d'isophoronenitrile selon au moins l'une quelconque des revendications précédentes, **caractérisé en ce que** le procédé est effectué dans un réacteur à lit fixe fonctionnant en continu.

19. Procédé pour la préparation d'isophoronenitrile selon au moins l'une quelconque des revendications précédentes, **caractérisé en ce que** le procédé est effectué dans deux phases au moyen d'un réacteur à lit fixe fonctionnant en continu pourvu d'un dispositif de présaturation.

20. Procédé pour la préparation d'isophoronenitrile selon au moins l'une quelconque des revendications précédentes 1-17, **caractérisé en ce qu'**un procédé en suspension (poudre en solution) est utilisé.

21. Utilisation d'éthanolate de calcium sous forme de particules, présentant une surface BET, mesurée au moyen d'un échantillon sous forme de poudre, d'au moins 5 m²/g et une densité apparente d'au moins 250 g/l, comme catalyseur pour la préparation d'isophoronenitrile à partir d'isophorone et d'acide cyanhydrique, la réaction étant effectuée en phase hétérogène.
